(19) Europäisches Patentamt
European Patent Office
Office européen des brevets

(11) Publication number: 0 313 165 B1

(12) # EUROPEAN PATENT SPECIFICATION

(45) Date of publication of patent specification: 13.01.93

(51) Int. Cl.5: C07C 37/20, C07C 39/16

(21) Application number: 88202330.2

(22) Date of filing: 18.10.88

(54) **A process for isomerizing the by-products of the bis-phenol synthesis.**

(30) Priority: 19.10.87 US 109894
19.10.87 US 111240

(43) Date of publication of application:
26.04.89 Bulletin 89/17

(45) Publication of the grant of the patent:
13.01.93 Bulletin 93/02

(84) Designated Contracting States:
DE FR GB NL

(56) References cited:
EP-A- 0 268 318
US-A- 4 294 995
US-A- 4 400 555

CHEMICAL ABSTRACTS, vol. 82, no. 18, 5th May 1975, page 13, abstract no. 112410h, Columbus, Ohio, US; & JP-A-74 20 565

CHEMICAL ABSTRACTS, vol. 96, no. 12, 22nd March 1982, page 10, abstract no. 86122h, Columbus, Ohio, US; & JP-A-81 142 227

(73) Proprietor: SHELL INTERNATIONALE RESEARCH MAATSCHAPPIJ B.V.
Carel van Bylandtlaan 30
NL-2596 HR Den Haag(NL)

(72) Inventor: Li, Simon Ming-kung
15318 El Padre Drive
Houston Texas 77083(US)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services

## Description

The present invention relates to the isomerization of by-products from the preparation of a bis-phenol.

Many processes are known to prepare bis-phenol-A. In some of these processes phenol is reacted with acetone to form bis-phenol-A. It is customary to then isolate the bis-phenol-A by crystallization, distillation or adduct crystallization. The concentrated residue contains many isomerizable components, such as o,p'-bis-phenol-A and other by-products from bis-phenol-A. These components of the residue are usually isomerized in an acidic medium to the desired bis-phenol-A. The acidic medium includes inorganic acids such as hydrochloric acid and acidic cation-exchange resins.

U.S. patent specification 3,221,061 discloses the preparation of bis-phenol-A and a subsequent "rearrangement" reaction conducted in the presence of a phenol saturated (mercapto alcohol modified) cation-exchange resin.

U.S. patent specification 4,400,555 discloses a multi-step synthesis in which acetone is injected in portions and an isomerization follows but the patent fails to illustrate the kind of specific catalyst in the isomerization zone.

U.S. patent specification 4,590,303 discloses a similar process in which the catalyst in the "rearrangement" reaction is a mercapto modified macroporous ion-exchange resin and the acetone is injected into the "rearrangement" reactor. While the total conversion of acetone was increased, the percentage of undesired by-products also increased. However, from the data in the experiments in this specification, it can be seen that diverting a part of the acetone to the "rearrangement" reactor was adverse to isomerization since the selectivity to the desired bis-phenol-A became progressively worse. Thus, the desired isomerization was not demonstrated.

U.S. patent specification 4,375,567 discloses that both microreticular and macroreticular ion-exchange resins in an unmodified form are used for isomerization. In this case, the microreticular resins were less effective for isomerization than the macroreticular resins.

There still exists a need to reduce or utilize the amount of undesirable by-products from the preparation of bis-phenols, e.g. bis-phenol-A from phenol and acetone. There is still a need to more effectively convert these undesired isomers into the desired bis-phenol. The present invention addresses this problem and provides a new method to isomerize the undesired by-products to the desired bis-phenol.

The present invention is directed to a process for isomerizing the by-products from the preparation of a bis-phenol in the desired 4.4'-dihydroxy form from a phenol and a ketone, which comprises treating the by-products either with a catalytic amount of an acid in the presence of a free mercaptan cocatalyst or with a catalytic amount of an acidic sulfonated cation-exchange resin having a plurality of sulfonic acid sites ionically bonded to an alkyl-mercaptoamine of the formula I

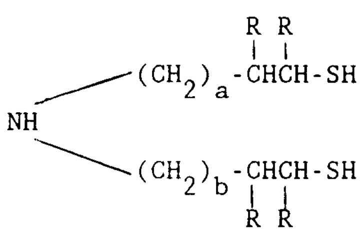

(I)

wherein each of a and b is an integer from 0 to 5 and each R individually represents H, OH, SH or alkyl and recovering a product having a higher concentration of the desired bis-phenol having the 4,4'-dihydroxy form.

The present invention is useful in recovering more of a desired bis-phenol, e.g. bis-phenol-A, by isomerization of an undesired bis-phenol such as 2,4'-dihydroxy-2,2-diphenyl propane (commonly referred to as o,p'-BPA) and related by-products to the desired bis-phenol-A with less formation of certain other undesirable impurities such as 1,3,3-trimethyl-p-hydroxyphenyl-6-hydroxyindane, and 4-methyl-2,4-bis-(4'-hydroxyphenyl)pentene-2.

The present invention is useful for the isomerization of certain undesirable by-products from the preparation of a bis-phenol from a ketone and a phenol. The bis-phenols include those prepared by the reaction of a ketone, such as acetone, ethyl methyl ketone, isobutyl methyl ketone, acetophenone, cyclohexanone and 1,3-dichloro acetone, with a phenol, such as phenol, o-cresol, m-cresol, o-chlorophenol, m-chlorophenol, o-t-butylphenol, 2,5-xylenol, 2,5-di-t-butylphenol and o-phenylphenol.

Suitable catalysts for the isomerization are alkylmercaptoamine-modified acidic sulfonated cation-exchange resins in which the alkyl-mercaptoamine according to formula I contains from 4 to 30 carbon atoms. Such resins which can be used as catalysts are conventionally known in the art and include those acidic cation-exchange resins that are modified by conventional procedures known in the art with an alkylmercaptoamine modifying agent of the invention either before use in the isomerization process or in some cases modified by the addition of the mercapto modifying agent to the isomerization process for use in conjunction with the previously unmodified acidic cation-exchange resin.

In the formula I, each of a and b is an integer from 0 to 5 and each R individually represents H, OH, SH or alkyl groups, preferably containing from 1 to 4 carbon atoms. As can be seen from the formula, the latter catalyst contains a high acidity density per given mercaptan concentration or a higher mercaptan concentration per given residual acidity density, as compared to cationic-exchange resin catalysts modified with monofunctional mercaptan alcohol promoters. The catalyst exhibits high activity and selectivity at moderate reaction temperatures, low production of coloured impurities and good stability.

The preparation of the aminomercaptan modifier of Formula I has been described in earlier copending U.S. patent application 925,779.

The currently preferred modified cationic exchange resin catalysts of the invention can be described by the Formulas II and III

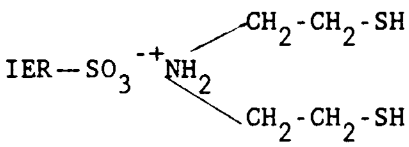

and

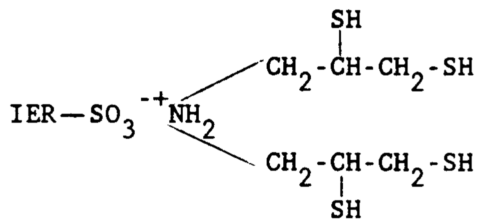

(II) (III)

in which IER represents the ion-exchange resin backbone.

The modified catalyst will generally have from 2 mole percent to 50 mole percent of mercapto-modified acidic groups, preferably from 3 mole percent to 25 mole percent.

The resins effectiveness in the mercapto modified resin isomerization process of the invention is to some extent influenced by their exchange capacities such that the greater the exchange capacity then the more desirable the resin is for isomerization. Preferably, the cation-exchange capacity is at least about 0.5 and, preferably greater than 4.0 meq/g dry weight. Also, those cation-exchange resins having bound cationic exchange groups of the stronger exchange potential acids are preferred for use in the mercapto modified resin isomerization process of the present invention. Acidic cation-exchange resins suitable for modification with a mercapto modifying agent for use in isomerization include sulfonated styrene-divinylbenzene copolymers, sulfonated cross-linked styrene polymers, phenol-formaldehyde-sulfonic acid resins, and benzene-formaldehyde-sulfonic acid resins. These include resins under such tradenames as Amberlites (Rohm and Haas Co.), DOWEX (Registered Trade Mark) (Dow Chemical Co.), Permutit QH (Permutit CO.), Chempro (Chemical Process Co.), Lewatit (Bayer A.G.) and the like. Strong acid sulfonated styrene-divinylbenzene copolymer resins are preferred. Commercially available aromatic sulfonic acid resins are generally obtained as sodium salts and are converted to the acid form prior to use. Both modified macroreticular resins and microreticular resins are useful in the isomerization process of the present invention. The choice of resin will of course depend on the material to be isomerized, the reaction conditions and the effect of an individual resin under the conditions selected, which determination and selection is within the skill of the art. Macroreticular resins are preferred.

Further any conventional acid which functions to isomerize undesired bis-phenol by-products to the desired bis-phenols can be used, together with a free mercaptan as a cocatalyst. These acids include both inorganic and organic acids or acidic acting materials, for example mineral acids, such as hydrochloric acid, hydrobromic acid, phosphoric acid or sulfuric acid, sulfonic acids such as toluene sulfonic acid, organic acids such as lower alkanoic acids having from 1 to 10 carbon atoms, acidic clays, boron trifluoride complexes or various acidic resins. The acid can be one formed by the hydrolysis with water of compounds such as aluminum chloride, sulfonyl chloride or phosgene. The acid can be a liquid or a solid but is preferably a solid itself or a solid used to carry or support acidic materials.

The catalysts for the isomerization are preferably acidic cation-exchange resins, as already mentioned hereinbefore. Such resins which can be used as catalysts are conventionally known in the art and include those acidic cation-exchange resins that are unmodified with a mercapto modifying agent before use in the

isomerization process, but which are used with the addition of a free mercaptan co-catalyst to the isomerization process.

The resin is essentially not modified to any substantial degree with the free mercaptan. The free mercaptan can be any free mercaptan of the type conventionally known in the art which includes any compound which will not react substantially under the isomerization conditions with the acid catalyst, including mercaptans, will not react with the acidic groups of the cation-exchange resin to introduce a mercaptan substituent into the resin. Suitable mercaptan co-catalysts include those of the formula $R'SH$ in which $R'$ is hydrogen or an organic group such as aliphatic, cycloaliphatic, aryl or heterocyclic compounds containing one or more free mercaptan groups. For convenience, the mercaptan usually is a non-resinous compound containing from 1-20 carbon atoms. Simple alkyl mercaptans, mercapto acids and precursors are used, for example, methyl mercaptan, dithioethane, ethyl mercaptan, n-pentyl mercaptan, thioglycolic acid or 1,2-dimercapto ethane. Alkyl mercaptans are preferred, especially methyl mercaptan. The amount of mercaptan present can vary with the acid used but is usually present in a lesser amount compared to the acid. For example, the mercaptan is present from 1 mole percent to 100 mole percent based on the acid and preferably from 5 to 50 moles percent.

The precise catalytic amount of acidic cation-exchange resin or of mercapto-modified acidic cation-exchange resin to be used will usually vary to some degree depending on the specific resin, feed and conditions used for isomerization process. By way of illustration, the catalyst can be present from 0.05 kg per kg of feed to 10.0 kg per kg of feed and preferably from 0.2 kg to 2 kg per kg of feed.

The isomerization is usually conducted in the presence of minor amounts of water in the reaction solution of from 1.5% by weight to essentially anhydrous conditions based on the isomerization reaction solution. Somewhat higher amounts of water can be present but this could result in less desirable rate of reaction. Somewhat higher amounts of water can also decrease net formation of the non-reversible impurities in the desired product. Preferably, the water content of the reaction solution is from 0.1% to 0.7% by weight based on the isomerization reaction solution.

The isomerization reaction is usually conducted at moderately elevated temperatures. Suitable temperatures are from 50 °C to 110 °C at ambient pressure. Preferably, the reaction temperature is from 60 °C to 85 °C at ambient pressure.

Thus, the isomerization reaction is conducted by contacting a feed stream containing liquid by-products from the preparation of a bis-phenol, such as 2,4′-dihydroxy-2,2-diphenyl propane and related by-products and optionally (phenol) washings from the crystallization of bis-phenol-A, with the catalyst under moderately elevated temperatures. The feed stream passes through the resin catalyst for a period of time sufficient to effect isomerization depending on the feed rate, size of the resin bed. The resulting isomerization product enriched in the desired bis-phenol having a 4,4′-dihydroxy form, such as bis-phenol-A, is then recovered. Usually the recovered product is recycled back to a zone in which the bis-phenol is prepared by condensation of a ketone (acetone) and phenol.

The reaction time in the isomerization or in the condensation depends on the reaction temperature and other reaction conditions, including whether the process is continuous or batch processing.

Another embodiment of the present invention is directed to an integrated process for the preparation of a bis-phenol having a desired 4,4′-dihydroxy form which comprises (a) condensing a ketone and a phenol in the presence of an acid, such as an acidic cation-exchange resin, (b) crystallizing an adduct of the phenol and the bis-phenol, and (c) isomerizing the by-products of the condensation step (a) optionally with any wash liquids from the crystallization step (b) in the presence of a catalyst.

The condensation of acetone and phenol can be conducted using cation-exchange resin conventionally known in the art for the condensation of acetone and phenol. In general, these are often mercapto modified resins of the type conventionally known in the art which include any compound which will react with the acidic groups of the cation-exchange resin to introduce a mercapto substituent into the resin. Suitable mercapto modifying agents include simple alkyl mercaptans, alkyl mercapto amines, mercapto alcohols, and precursors, for example, methyl mercaptan, propylaminopropyl mercaptan, mercaptan, bis-2-(mercaptoethyl)-amine, mercapto ethanol, thiazolidine, although unmodified resins are also useful.

The condensation reaction is conducted at moderately elevated temperature of from 50 °C to 130 °C at ambient pressures.

In the preparation of the bis-phenols, an excess of the phenol is usually desirable, generally from 5 to 20 moles of phenol per mole of ketone, is desirable for high conversion of the ketone. Solvents or diluents are not necessary in either the preparation of the bis-phenol or in the isomerization of the undesired by-product except at low temperature.

The bis-phenol product, e.g., bis-phenol-A, is passed to a concentrator where the acetone and phenol and excess water are removed as an overhead fraction. The crude bis-phenol-A product is then passed to a

crystallization zone where it is chilled to a temperature between 30 °C and 95 °C to form an adduct of phenol and bis-phenol-A which separates out as crystals. After washing with phenol, filtering and the like, the bis-phenol-A is recovered from the adduct. The mother liquid by-product stream from the crystallization zone is passed to the isomerization zone, optionally combined with the phenol washings from the crystallization step, and isomerized in the presence of a catalyst as described above. The product of this isomerization enriched in bis-phenol-A is recovered or preferably recycled to the condensation zone.

An integrated process for bis-phenol-A may be described briefly as follows: Acetone and phenol are injected into a condensation reactor along with any recycle isomerization product. Any conventional condensation catalyst effective for the formation of bis-phenol-A can be used. However, it is advantageous that the reactor contains an unmodified or modified cation-exchange resin, such as a macroreticular sulfonated polystyrene divinylbenzene acidic cation-exchange resin at 50 °C to 90 °C. The reaction product is passed into a concentrator in which unreacted acetone and phenol and excess water are recovered for recycle to the condensation reactor. Crude bis-phenol-A product is recovered and passed to a crystallizer to form a solid bis-phenol-A/phenol adduct. The slurry is passed into separator wherein the adduct is separated from the by-product mother liquid and is passed into a melter. The by-product mother liquid is removed from the separator and passed into an isomerization zone. The isomerization zone is maintained at 60 °C to 90 °C and contains an acidic cation-exchange resin which is used in a mercapto modified form. This is conveniently a microreticular or macroreticular acidic cation-exchange resin such as sulfonated styrene-divinylbenzene modified with 10% of bis-2-(mercaptoethyl)-amine. The isomerization product which is increased in concentration of the desired bis-phenol-A is recycled to the condensation reactor.

While the invention has been illustrated with particular apparatus, those of skill in the art will appreciate that equivalent or analogous apparatus or parts thereof can be employed and that the use of equipment operated in series of in parallel can be used. Batch of continuous form can be used. The solid catalysts can be used as a slurry with the reactants in batch processing or in a fixed bed in a continuous process.

The invention is illustrated by the following examples which should not be regarded as limiting the invention in any way.

EXAMPLES 1 to 3

Experiments were performed in batch at 80 °C using a 1:3 catalyst to reactant weight ratio for the following systems: 1) unmodified macroporous DOWEX MSC-1; 2) 10% bis-2-(mercaptoethyl)-amine (BMEA) modified macroporous DOWEX MSC-1; and 3) 1% by weight n-pentyl mercaptan with unmodified macroporous DOWEX MSC-1. Each catalyst was pre-dried in vacuum oven at 60 °C-70 °C for 1-2 days and the reactant source was by-product from bis-phenol-A obtained by the condensation of acetone and phenol containing undesired 2,4′-dihydroxy-2,2-diphenyl propane. CDB (1,3,3-trimethyl-p-hydroxyphenyl-6-hydroxyindane) and LDP-1 (4-methyl-2,4-bis-(4'-hydroxyphenyl)pentene-2) were undesirable by-products of isomerization with unmodified catalysts and were also present in the isomerization feed. Results are given in the Table.

Table

| System[a)] | %w Water[b)] | Δ CDB/LDP-1[c)] (ppm) | Δ Heavies[c)] or Unknown (ppm) |
|---|---|---|---|
| 1 | 0.22 | 5000 | 250 |
| | 0.35 | 1500 | 50 |
| 2 | 0.36 | 0 | 0 |
| 3 | 0.37 | 500 | 0 |

a) System: 1. Unmodified macroporous DOWEX MSC-1.
2. 10%-BMEA modified macroporous DOWEX MSC-1.
3. Unmodified macroporous DOWEX MSC-1 and 1 %w n-pentyl mercaptan.

b) Water in solution phase.

c) Net increase at 80 °C after 50% of isomerizable 2,4'-dihydroxy-2,2-diphenyl propane has been converted to 4,4'-dihydroxy-2,2-diphenyl propane.

EXAMPLE 4

Following procedures similar to those described in Example 1, by-products from the preparation of bis-phenol-A were isomerized with a 15% bis-2-(mercaptoethyl)-amine modified microreticular DOWEX 50WX2 resin in the presence 0.35% w water in the reactant solution. No Δ CDB/LDP-1 was obtained after 50% of the isomerizable o,p′-BPA has been converted to p,p′-BPA, although some heavies and a new impurity were obtained.

## Claims

1. A process for isomerizing the by-products from the preparation of a bis-phenol obtained from the condensation of a ketone and a phenol and which comprises treating the by-products either with a catalytic amount of an acid in the presence of a free mercaptan cocatalyst or with a catalytic amount of a sulfonated cation-exchange resin having a plurality of sulfonic acid sites ionically bonded to an alkylmercaptoamine of the Formula I

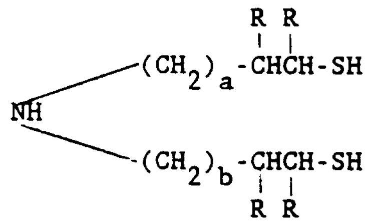

(I)

wherein each of a and b is an integer from 0 to 5 and each R individually represents H, OH, SH or alkyl and recovering a product having a higher concentration of the desired bis-phenol having the 4,4'-dihydroxy form

**2.** A process according to claim 1 wherein the cation-exchange resin has the Formula II or III

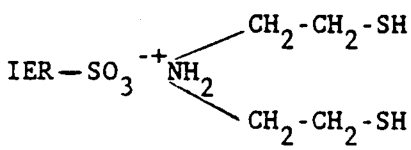

(II)

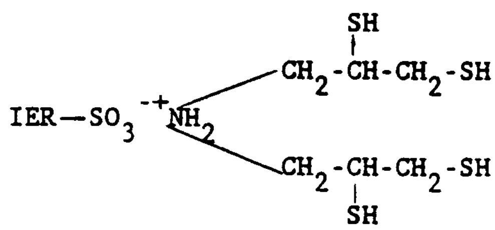

(III)

in which IER represents the ion-exchange resin backbone.

**3.** A process according to claims 1 or 2 wherein the cation-exchange resin is selected from sulfonated styrene-divinylbenzene copolymers, sulfonated cross-linked styrene polymers, phenol-formaldehyde-sulfonic acid resins and benzene-formaldehyde-sulfonic acid resins.

**4.** A process according to claim 1 wherein the acid (in the presence of a free mercaptan cocatalyst) is a cation-exchange resin.

**5.** A process according to claim 4 wherein the cation-exchange resin is selected from sulfonated styrene-divinylbenzene copolymers, sulfonated cross-linked styrene polymers, phenol-formaldehyde-sulfonic acid resins and benzene-formaldehyde-sulfonic acid resins.

**6.** A process according to claim 1, 4 or 5 wherein the cocatalyst is an alkyl mercaptan.

**7.** A process according to claim 6 wherein the cocatalyst is n-pentyl mercaptan.

**8.** A process according to claim 1 wherein the by-products are from the preparation of bis-phenol-A and comprise 2,4'-dihydroxy-2,2-diphenyl propane.

**9.** A process according to any one of the claims 1-8 wherein the isomerization is carried out at a temperature from 50 °C to 110 °C.

**10.** An integrated process for the preparation of a bis-phenol which comprises (a) condensing a ketone and a phenol in the presence of an acidic cation-exchange resin, (b) crystallizing an adduct of the phenol and the bis-phenol, and (c) isomerizing the by-products of the condensation step (a) optionally with any wash liquids from the crystallization step (b) according to claim 1.

## Patentansprüche

**1.** Ein Verfahren zur Isomerisierung der Nebenprodukte, welche bei der Herstellung eines Bisphenols, erhalten durch Kondensation eines Ketons und eines Phenols, anfallen, und welches Verfahren die

Behandlung der Nebenprodukte entweder mit einer katalytischen Menge einer Säure in Gegenwart eines freien Mercaptan-Cokatalysators oder mit einer katalytischen Menge eines sulfonierten Kationenaustauscherharzes mit einer Vielzahl von Stellen, an denen die Sulfonsäuregruppen ionisch an ein Alkylmercaptoamin der nachstehenden Formel I gebunden sind,

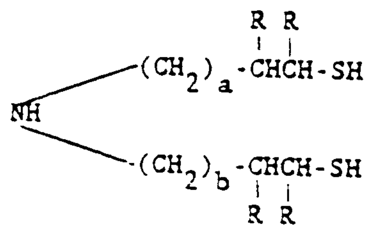

(I)

in welcher jedes a und jedes b eine ganze Zahl von 0 bis 5 bedeutet und jedes R individuell H, OH, SH oder Alkyl ist, und das Gewinnen eines Produktes mit einer höheren Konzentration an dem gewünschten Bisphenol in der 4,4'-Dihydroxyform umfaßt.

**2.** Ein Verfahren nach Anspruch 1, in welchem das Kationenaustauscherharz die nachstehenden Formeln II oder III aufweist.

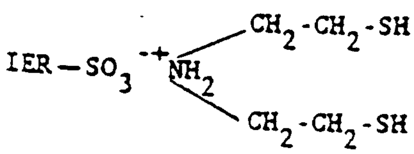

(II)

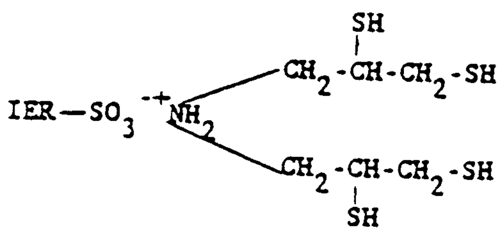

(III)

in welchen Formeln IER die Hauptkette des Ionenaustauscherharzes darstellt.

**3.** Ein Verfahren nach Anspruch 1 oder 2, in welchem das Kationenaustauscherharz ausgewählt ist aus sulfonierten Styrol-Divinylbenzol-Copolymerisaten, sulfonierten vernetzten Styrolpolymeren, Phenol-Formaldehyd-Sulfonsäureharzen und Benzol-Formaldehyd-Sulfonsäureharzen.

**4.** Ein Verfahren nach Anspruch 1, in welchem die Säure (in Anwesenheit eines freien Mercaptan-Cokatalysators) ein Kationenaustauscherharz ist.

**5.** Ein Verfahren nach Anspruch 4, in welchem das Kationenaustauscherharz ausgewählt ist aus sulfonierten Styrol-Divinylbenzol-Copolymeren, sulfonierten vernetzten Styrolpolymeren, Phenol-Formaldehyd-Sulfonsäureharzen und Benzol-Formaldehyd-Sulfonsäureharzen.

**6.** Ein Verfahren nach Anspruch 1, Anspruch 4 oder Anspruch 5, in welchem der Cokatalysator ein Alkylmercaptan ist.

**7.** Ein Verfahren nach Anspruch 6, in welchem der Cokatalysator n-Pentylmercaptan ist.

**8.** Ein Verfahren nach Anspruch 1, in welchem die Nebenprodukte aus der Herstellung von Bisphenol-A stammen und 2,4'-Dihydroxy-2,2-diphenylpropan enthalten.

**9.** Ein Verfahren nach irgendeinem der Ansprüche 1 - 8, wobei die Isomerisierung bei einer Temperatur im Bereich von 50 bis 110 ° C durchgeführt wird.

**10.** Ein integriertes Verfahren zur Herstellung eines Bisphenols, umfassend die folgenden Verfahrensstufen:

a) Das Kondensieren eines Ketons und eines Phenols in Anwesenheit eines sauren Kationenaustauscherharzes,

b) das Auskristallisieren eines Adduktes aus dem Phenol und dem Bisphenol, und

c) das Isomerisieren der in der Kondensationsstufe (a) anfallenden Nebenprodukte, gegebenenfalls zusammen mit in der Kristallisationsstufe (b) anfallenden Waschflüssigkeiten, gemäß der Arbeitsweise von Anspruch 1.

**Revendications**

**1.** Procédé d'isomérisation des sous-produite de la préparation d'un bisphénol, obtenu par la condensation d'une cétone et d'un phénol, qui comprend le traitement des sous-produits soit avec une quantité catalytique d'un acide en présence d'un cocatalyseur mercaptan libre, soit avec une quantité catalytique d'une résine échangeuse de cations sulfonée ayant plusieurs sites d'acide sulfonique unis par liaison ionique à une alkylmercaptoamine

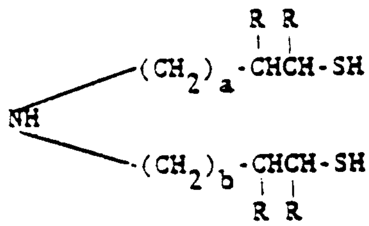

(I)

dans laquelle chacun de a et de b est un entier de 0 à 5 et chaque R individuellement représente H, OH, SH ou un alkyle, et la récupération d'un produit ayant une concentration supérieure du bisphénol désiré ayant la forme 4,4'-dihydroxy.

**2.** Procédé selon la revendication 1, dans lequel la résine échangeuse de cations a la formule II ou III

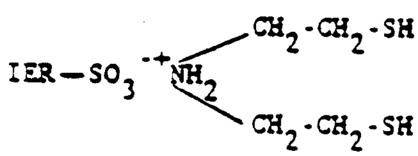

(II)

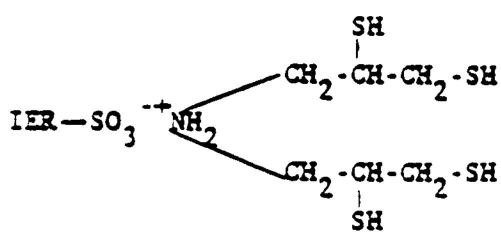

(III)

où IER représente la charpente de la résine échangeuse d'ions.

**3.** Procédé selon les revendications 1 ou 2, dans lequel la résine échangeuse de cations est choisie parmi les copolymères de styrène-divinylbenzène sulfonés, les polymères de styrène réticulés et sulfonés, les résines de phénol-formaldéhyde-acide sulfonique et les résines de benzène-formaldéhyde-acide sulfonique.

**4.** Procédé selon la revendication 1, dans lequel l'acide (en présence d'un cocatalyseur mercaptan libre) est une résine échangeuse de cations.

**5.** Procédé selon la revendication 4, dans lequel la résine échangeuse de cations est choisie parmi les copolymères de styrène-divinylbenzène sulfonés, les polymères de styrène réticulés et sulfonés, les résines de phénol-formaldéhyde-acide sulfonique et les résines de benzène-formaldéhyde-acide sulfonique.

**6.** Procédé selon les revendications 1, 4 ou 5, dans lequel le cocatalyseur est un alkylmercaptan.

**7.** Procédé selon la revendication 6, dans lequel le cocatalyseur est le n-pentylmercaptan.

**8.** Procédé selon la revendication 1, dans lequel les sous-produits proviennent de la préparation du bisphénol A et comprennent du 2,4'-dihydroxy-2,2-diphénylpropane.

**9.** Procédé selon l'une quelconque des revendications 1 à 8, dans lequel l'isomérisation est effectuée à une température de 50 ° C à 110 ° C.

**10.** Procédé intégré pour la préparation d'un bisphénol, qui comprend (a) la condensation d'une cétone et d'un phénol en présence d'une résine échangeuse de cations acide, (b) la cristallisation d'un produit d'addition du phénol et du bisphénol et (c) l'isomérisation des sous-produits de l'étape de condensation (a) éventuellement avec des liquides de lavage de l'étape de cristallisation (b) selon la revendication 1.